# FASCICULE DE BREVET EUROPEEN

(11) **EP 0 744 974 B1**
(45) Date de publication et mention de la délivrance du brevet: **10.06.1998**
(21) Numéro de dépôt: 95909828.6
(22) Date de dépôt: 15.02.1995
(51) Int. Cl.: A61M 5/172

(54) **INSTALLATION DE SURVEILLANCE A DISTANCE D'EQUIPEMENTS COMMANDABLES**
ANORDNUNG ZUR FERNÜBERWACHUNG VON STEUERBAREN EINRICHTUNGEN
APPARATUS FOR REMOTELY MONITORING CONTROLLABLE DEVICES

(30) Priorité: 16.02.1994 FR 9401747
(43) Date de publication de la demande: 04.12.1996
(73) Titulaire: DEBIOTECH S.A., 1000 Lausanne 9 (CH)
(72) Inventeur: NEFTEL, Frédéric, CH-1000 Lausanne 9 (CH)
(74) Mandataire: Dronne, Guy
(86) Numéro de dépôt international: FR9500175
(87) Numéro de publication internationale: WO9522363

(56) Documents cités:
- WO-A-92/08207
- WO-A-93/01574
- DE-A- 3 036 217
- DE-A- 4 127 014
- DE-A- 4 235 816
- US-A- 3 767 859
- NINTH ANNUAL CONFERENCE OF THE IEEE ENGINEERING IN MEDICINE AND BIOLOGY SOCIETY, 16 Novembre 1987, BOSTON US pages 230 - 231 S. SUBRAMANIAN ET AL 'Toward a totally integrated hospital information system: Infusion pump link to patient data management system'

## Description

La présente invention a pour objet une installation de surveillance à distance d'une pluralité d'équipements commandables, notamment d'équipements utilisables dans des centres hospitaliers, tels qu'une pompe de perfusion ou d'administration de médicaments.

De façon plus précise, l'invention concerne une installation qui permet de surveiller à distance de façon sécurisée des équipements, en particulier des équipements hospitaliers d'administration de liquides injectables à des patients qui permette en même temps d'exercer un contrôle permanent à distance du mode de fonctionnement de ces équipements et du stade de fonctionnement de ceux-ci et également, de préférence mais non nécessairement, de les commander à distance.

On comprend que, dans un centre hospitalier, les différents équipements à contrôler, notamment les pompes pour l'administration de médicaments sont situés dans des locaux différents et destinés à des malades différents, une seule personne étant chargée du contrôle du bon fonctionnement de ceux-ci. Il est donc intéressant de fournir à la personne chargée du contrôle du fonctionnement des équipements un moyen pour pouvoir savoir en permanence où en est le fonctionnement de chacun des équipements.

Il est également préférable que la possibilité d'accès à la commande de l'équipement pour en modifier éventuellement la programmation soit strictement limitée à la personne chargée du fonctionnement ou au moins soit limitée à un nombre réduit de personnes autorisées.

Il est également souhaitable, dans ce type d'installation de commande à distance de pouvoir savoir, en cas d'erreur de programmation ou de commande d'un équipement ayant entraîné des conséquences dommageables, par exemple pour un patient, qui, parmi le personnel de l'établissement hospitalier, est responsable de cette mauvaise programmation.

Un objet de la présente invention est de fournir un dispositif de surveillance à distance d'une pluralité d'équipements commandables, notamment d'équipement rencontré dans un centre hospitalier ou similaire qui permette une surveillance continue à distance de l'évolution du fonctionnement des équipements et de préférence qui permette de limiter aux personnes autorisées la possibilité de programmer ou de modifier la programmation de ces équipements.

Pour atteindre ce but, selon l'invention, l'installation de surveillance à distance d'une pluralité d'équipements commandables, chaque équipement comprenant des moyens de commande activés en réponse à une séquence d'informations de commande incluant une information de durée de fonctionnement, se caractérise en ce qu'elle comprend un dispositif portatif de commande et/ou surveillance détenu par le préposé à la surveillance comportant :
. des moyens de mémorisation pour mémoriser au moins une partie des informations de commande relatives à chaque équipement en relation avec une information d'identification dudit équipement, lesdites informations mémorisées comprenant au moins l'information représentative de la durée commandée de fonctionnement dudit équipement,
. des moyens pour élaborer une information de temps,
. des moyens pour initialiser l'information de temps en relation avec la commande du début de fonctionnement dudit équipement et des moyens de mesure de temps par quoi une information de temps écoulé est obtenue, et
. des moyens pour comparer ladite durée de fonctionnement à l'information de temps écoulé et pour émettre un signal d'alarme en fonction du résultat de ladite comparaison.

De préférence, l'installation comprend en outre des moyens pour n'autoriser l'introduction d'une séquence d'informations de fonctionnement dans un équipement qu'en réponse à une information spécifique.

On comprend que, grâce à une telle installation de surveillance à distance, la personne ou les personnes préposées à l'utilisation des équipements connaît(ssent) en permanence, grâce au dispositif portatif qu'elle(s) détient(nent) le temps depuis lequel un équipement ou chaque équipement fonctionne et donc le temps de fonctionnement restant. De plus, cette (ou ces) personne(s) sont informées de la fin du fonctionnement de l'équipement, ce qui permet à cette personne de se déplacer éventuellement pour contrôler visuellement l'arrêt de l'équipement et prendre éventuellement auprès du patient les dispositions requises. Il faut également préciser que, par l'expression "une information représentative de la durée de fonctionnement de l'équipement", il faut entendre soit une information de durée effectivement de fonctionnement, par exemple dans le cas d'une pompe, soit, par exemple dans le cas d'un équipement de goutte à goutte, une information de volume à administrer qui aura été convertie en un équivalent de temps par une mesure initiale du fonctionnement de l'équipement de goutte à goutte.

Selon un premier mode de mise en oeuvre, les moyens de commande de chaque équipement comprennent des moyens pour transmettre la séquence d'informations de commande de fonctionnement et en ce que ledit dispositif portatif comprend des moyens pour recevoir ladite séquence d'informations.

Dans ce mode de mise en oeuvre, les équipements sont directement commandés par le préposé. Les séquences d'information de commande de fonctionnement sont automatiquement transmises au dispositif portatif détenu par le préposé et elles y sont automatiquement mémorisées, ce qui évite tout risque d'erreur.

Selon un deuxième mode de mise en oeuvre, l'installation de surveillance comprend en outre :
- une pluralité de dispositifs récepteurs desdites informations de commande, chaque dispositif récepteur étant monté sur un desdits équipements, ledit dispositif portatif de commande comprenant en outre :
   . des moyens pour élaborer ladite séquence d'informations de commande de fonctionnement d'un équipement ; et
- des moyens pour transmettre vers un dispositif récepteur ladite séquence d'informations de commande, chaque dispositif récepteur comportant :
   . des moyens de réception desdites informations de commande transmises, et
   . des moyens pour commander ledit équipement en fonction desdites informations.

Dans ce deuxième mode de mise en oeuvre, le dispositif portatif sert, d'une part, à transmettre les informations de commande vers les dispositifs récepteurs associés aux équipements et, d'autre part, à mémoriser ces informations de commande et à contrôler la durée du fonctionnement de l'équipement.

Selon un mode préféré de mise en oeuvre, la séquence d'informations de commande comprend une information d'identification de l'équipement commandé et chaque dispositif récepteur comprend en outre des moyens de mémorisation d'une information d'identification de l'équipement sur lequel il est monté, des moyens pour comparer l'information d'identification mémorisée à l'information d'identification reçue et des moyens pour n'autoriser la mise en oeuvre d'au moins certaines des autres informations reçues que s'il y a correspondance entre les deux informations d'identification.

Selon une variante de mise en oeuvre, la séquence d'informations comprend en outre une information d'identification du dispositif portatif de commande et chaque dispositif récepteur comprend des moyens pour mémoriser l'information d'identification du dispositif portatif de commande des moyens pour comparer l'information d'identification mémorisée à l'information d'identification reçue ultérieurement et des moyens pour n'exécuter au moins certaines desdites informations que s'il y a correspondance entre les deux informations d'identification.

On comprend qu'avec ces deux modes de mise en oeuvre préférés, il est possible de contrôler l'introduction dans les moyens de commande de chaque équipement des informations de fonctionnement ou des informations de modifications du fonctionnement, ce qui protège les équipements contre des introductions d'instructions de commande erronées. En d'autres termes, cela permet de n'autoriser qu'un nombre limité de personnes à intervenir sur le fonctionnement des équipements commandés.

Selon également un mode préféré de mise en oeuvre, chaque dispositif récepteur comprend en outre des moyens émetteurs pour retransmettre au moins une partie de la séquence d'informations de commande reçues et ledit dispositif portatif de commande comporte des moyens de réception des informations réémises, des moyens pour comparer les informations réémises aux informations émises et des moyens pour émettre un signal d'acquittement si les informations émises et les informations réémises correspondent entre elles.

Selon ce mode préféré de mise en oeuvre, on voit qu'il est possible de s'assurer que la transmission d'informations de commande entre le dispositif portatif de commande et les dispositifs récepteurs associés à chaque équipement a été correctement décodé par les dispositifs récepteurs des équipements, ce qui permet bien sûr de s'affranchir de tout risque d'erreur résultant de la transmission à distance des séquences d'informations. Cela permet également d'initialiser le décompte du temps de fonctionnement.

D'autres caractéristiques et avantages de la présente invention apparaîtront mieux à la lecture de la description qui suit de plusieurs modes de réalisation de l'invention donnés à titre d'exemples non limitatifs. La description se réfère aux figures annexées sur lesquelles :
- la figure 1 est une vue simplifiée et externe d'un dispositif de surveillance portatif ;
- la figure 2 est une vue simplifiée d'un premier mode de réalisation d'une installation de surveillance et de commande à distance ;
- la figure 3 est un schéma montrant les circuits internes du dispositif de surveillance et de commande à distance de la figure 1 ;
- la figure 4 montre partiellement une variante de réalisation de l'installation de surveillance et de commande à distance ; et
- la figure 5 est une vue simplifiée d'un autre mode de mise en oeuvre d'une installation permettant seulement la surveillance à distance.

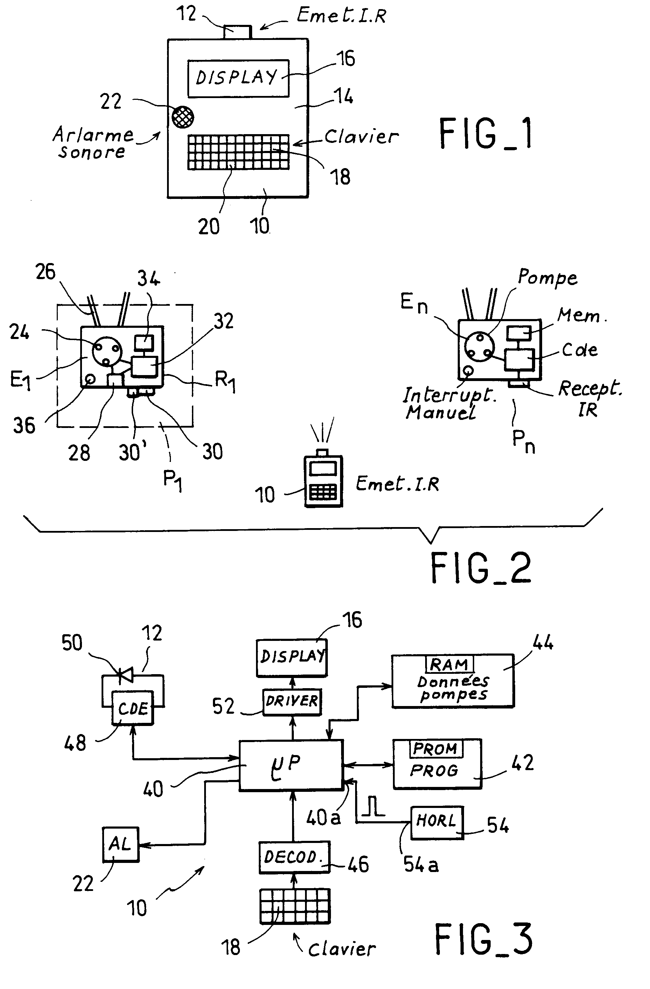

En se référant tout d'abord aux figures 1 à 4, on va décrire un mode de mise en oeuvre de l'invention qui permet à la fois la commande et la surveillance à distance des équipements.

En se référant à la figure 1, on va décrire l'aspect extérieur du dispositif de commande portatif. Celui-ci se présente de façon classique, sous la forme d'un boîtier 10 qui comporte un émetteur de rayonnement électromagnétique et, de préférence, de rayonnement infrarouge. De préférence, le boîtier comporte de plus un récepteur de rayonnement qui peut d'ailleurs être confondu avec l'émetteur. Sur sa face avant 14, le boîtier 10 comprend un écran d'affichage 16, par exemple à cristaux liquides, et un clavier 18 constitué par des touches 20 permettant d'entrer des informations dans le dispositif de commande. Le boîtier comporte de plus, de préférence, un dispositif 22 d'alarme sonore et/ou lumineuse et/ou vibratoire.

La figure 2 illustre sous une forme schématique l'ensemble de l'installation de commande à distance des équipements selon un premier mode de réalisation. Cette installation comporte au moins un dispositif de commande 10 qui est détenu par une personne chargée de la gestion du fonctionnement des équipements et une pluralité d'équipements à commander et à surveiller E1....En. Dans l'exemple considéré, les équipements E1 à En sont des pompes par exemple du type péristaltique à galets 24 qui servent à administrer à un patient un médicament via une tubulure 26. Dans l'exemple de mise en oeuvre, chaque équipement Ei est installé dans une pièce P1....PN. Chaque équipement Ei est muni d'un circuit de commande 28 de l'équipement associé et d'un ensemble de réception et de traitement d'informations de fonctionnement. Chaque ensemble récepteur R1, R2.... comprend essentiellement un récepteur de rayonnement électromagnétique, de préférence de faisceau infrarouge 30 qui est relié à un circuit de traitement 32 associé de préférence à une mémoire ré-inscriptible 34. Chaque ensemble récepteur Ri peut avantageusement comprendre en outre un émetteur de faisceau infrarouge 30'. Le circuit de traitement 32 de type en soi connu est capable de décoder les signaux reçus par le détecteur 30 pour les transformer en instructions de commande du fonctionnement de l'équipement E1 et donc d'appliquer des signaux de commande au circuit de commande 28 de l'équipement. En outre, de préférence, l'équipement E1 comporte un organe d'arrêt d'urgence manuel 36 qui permet d'arrêter le fonctionnement de l'équipement dès qu'il est actionné en l'absence de la télécommande.

En se référant maintenant à la figure 3, on va décrire plus en détails les différents circuits du dispositif de commande E10 ainsi que les différentes fonctions que ce dispositif peut mettre en oeuvre. Le circuit du dispositif de commande est essentiellement constitué par exemple par un microprocesseur 40 associé d'une part à une mémoire de programme 42 qui est, par exemple, une mémoire du type EEPROM et à une mémoire de données 44 qui est, par exemple, une mémoire du type RAM ou similaire.

Le microprocesseur 40 peut recevoir des instructions qui sont élaborées par l'utilisateur à l'aide du clavier 18. Le décodeur 46 convertit les signaux fournis par l'actionnement des touches du clavier en signaux numériques de commande directement utilisables par le microprocesseur. Le microprocesseur 40 est également relié à une cellule émettrice/réceptrice 12 de faisceau infrarouge. Plus précisément, le microprocesseur 40 est relié à un circuit de commande et de codage 48 qui commande l'émission par la diode 50 lorsque celle-ci doit fonctionner en émetteur et qui sert à la conversion analogique/numérique des signaux reçues par la cellule 50 lorsque celle-ci fonctionne en récepteur. Le microprocesseur est également relié au circuit d'affichage 16 par l'intermédiaire d'un circuit de codage 52 bien connu en lui-même. Les circuits du dispositif de commande 10 comportent également un circuit d'horloge ou base de temps 54 qui délivre sur sa sortie 54a des impulsions de temps appliquées à l'entrée 40a du microprocesseur 40. Enfin, une des sorties, de préférence analogique, du microprocesseur est relié au dispositif d'alarme sonore 22.

On va maintenant expliquer l'utilisation du dispositif de commande 10 pour programmer le fonctionnement des équipements E1....EN associé au dispositif récepteur R1.....RN. Pour programmer un équipement, le détenteur du dispositif de commande 10 entre à l'aide du clavier 18 des instructions de fonctionnement de l'équipement à commander. Cette séquence d'instructions comporte nécessairement une information représentative de durée de fonctionnement qui peut être, comme on l'a déjà explique, une information de durée effective ou une information de volume à administrer, de préférence une information d'identification de l'équipement commandé et une information d'identification du dispositif de commande. Cette séquence d'informations peut comporter en outre des instructions concernant le régime de fonctionnement de l'équipement lorsqu'il s'agit d'une pompe ou encore d'autres consignes de fonctionnement. Ces instructions sont reçues par le microprocesseur 40 qui en commande l'affichage successif sur le circuit d'affichage 16 afin que l'utilisateur puisse en vérifier visuellement l'exactitude.

En réponse à ces instructions, le microprocesseur commande l'enregistrement de ces instructions dans la mémoire de données 44 et l'activation du circuit de commande 48 pour que l'émetteur infrarouge 50 émette un signal codé représentatif de ces différentes instructions. Ce signal infrarouge est reçu par le récepteur 30 de l'équipement E1 à commander. De préférence, le circuit de commande 32 de l'équipement qui est constitué essentiellement par un microprocesseur commande la réémission par l'émetteur 30' de la séquence d'instructions reçues vers le dispositif de commande. Ces informations réémises sont reçues par le détecteur 50 et le microprocesseur vérifie la conformité des informations émises et des informations reçues. En cas de concordance, il commande l'émission par l'émetteur 50 d'un signal d'acquittement vers l'équipement E1. A la réception de ce signal d'acquittement, le circuit de traitement 32 de l'équipement transmet vers le circuit de commande 28 les instructions de fonctionnement de l'équipement, par exemple de la pompe 24 associée.

En variante, il est possible de prévoir deux procédures permettant de s'assurer que les instructions transmises le sont licitement, c'est-à-dire par une personne autorisée. Selon un premier mode de contrôle, la mémoire 34 associée au circuit de traitement 32 comporte une information spécifique à l'équipement. Le circuit de traitement 32 compare cette information à l'information correspondante dans la séquence d'informations de commande reçue du dispositif de commande 10. Lorsqu'il y a correspondance, les instructions seront effectivement transmises au circuit de commande 28 de l'équipement associé. Selon la variante de contrôle, le circuit de mémoire 34 comporte une instruction d'identification du dispositif de commande et la séquence d'information émise par le dispositif portatif 10 comporte également une information d'identification. Le circuit de traitement 32 compare deux informations d'identification et des instructions de commande de fonctionnement de l'équipement ne seront exécutées que s'il y a concordance entre les deux informations d'identification.

L'émission par le dispositif de commande du signal d'acquittement provoque l'exécution par le microprocesseur 40 d'un sous-programme de décompte de temps qui permet de comparer l'information de temps s'écoulant fournie par la base de temps 54 à l'instruction de durée de fonctionnement de l'équipement qui est stockée dans la mémoire 44 en relation avec une information d'identification de l'équipement. Lorsque le sous-programme de comparaison détecte que le temps effectivement écoulé devient égal à la durée de fonctionnement de l'équipement, le microprocesseur 40 provoque l'excitation du dispositif d'alarme 22. Selon une variante de mise en oeuvre, il est possible de prévoir que le sous-programme de comparaison élabore un premier signal de comparaison lorsque le temps écoulé correspond à la durée programmée de fonctionnement diminuée d'une durée de pré-alarme prédéterminée et mémorisée dans la mémoire 42, cette durée pouvant être égale par exemple à 5 minutes. A la détection de l'instant de pré-alarme, le composant 22 émet un signal spécifique pour informer l'utilisateur que la fin du fonctionnement de l'équipement concerné E1 va bientôt intervenir. Cet équipement est identifié dans la mémoire du dispositif portatif par un numéro de chambre, un numéro de lit, un numéro de pompe, etc.

L'installation de commande à distance d'équipement qui vient d'être décrite présente de nombreux avantages par rapport à la commande manuelle de ces équipements.

Le préposé à la gestion d'un ensemble d'équipement qui détient le dispositif portatif de commande peut connaître à tout instant les paramètres de fonctionnement de chaque équipement dont il a la charge puisque ces paramètres sont stockés dans la mémoire du dispositif. Il peut donc les modifier en cas de besoin en émettant à l'aide de son dispositif de nouvelles valeurs de paramètres vers l'équipement concerné. En particulier, il peut savoir à tout instant la durée restante de fonctionnement de chaque équipement. En outre, il est automatiquement informé dès que le fonctionnement d'un équipement a cessé.

Grâce aux informations d'identification du dispositif de commande et des équipements, il est possible de n'autoriser que la détenteur d'un dispositif de commande donné à intervenir sur un équipement donné ou plutôt un groupe d'équipements donné.

Enfin, comme les instructions de fonctionnement des équipements restent dans la mémoire du dispositif de commande, en cas de problème, il est possible de savoir qui a donné les instructions de fonctionnement puisqu'il s'agit du détenteur du dispositif de commande dans la période de temps concerné. En outre, en transmettant le dispositif portatif à une autre infirmière, cette dernière dispose, sans risque d'erreur, de l'ensemble des informations relatives aux équipements dont elle va avoir la charge.

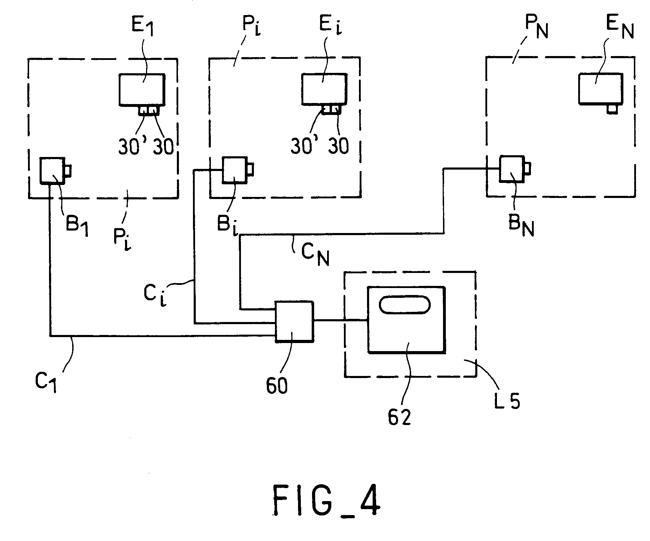

La figure 4 illustre un mode perfectionné de réalisation de l'installation de commande à distance selon le premier mode de réalisation. Sur cette figure, on a représenté les équipements Ei avec leurs dispositifs récepteurs Ri, chaque dispositif récepteur étant muni d'un récepteur infrarouge 30 et d'un émetteur infrarouge 30'. Chaque équipement Ei est installé dans un local Pi. Chaque local Pi est équipé d'une borne réceptrice de signaux infrarouges Bi capables de recevoir les signaux infrarouges émis par l'émetteur 30' de l'équipement Ei. Chaque borne Bi est reliée par un conducteur électrique Ci à un concentrateur 60 lui-même relié à un pupitre de télésurveillance 62 installé dans un local de surveillance LS. Selon ce mode de réalisation les circuits 28 et 32 des dispositifs récepteurs Ri sont configurés pour provoquer l'émission par l'émetteur 30' d'un signal infrarouge d'alarme en cas de mauvais fonctionnement de l'équipement auquel il est associé. Ce signal d'alarme est reçu par la borne Bi et transmis au pupitre 62 de télésurveillance.

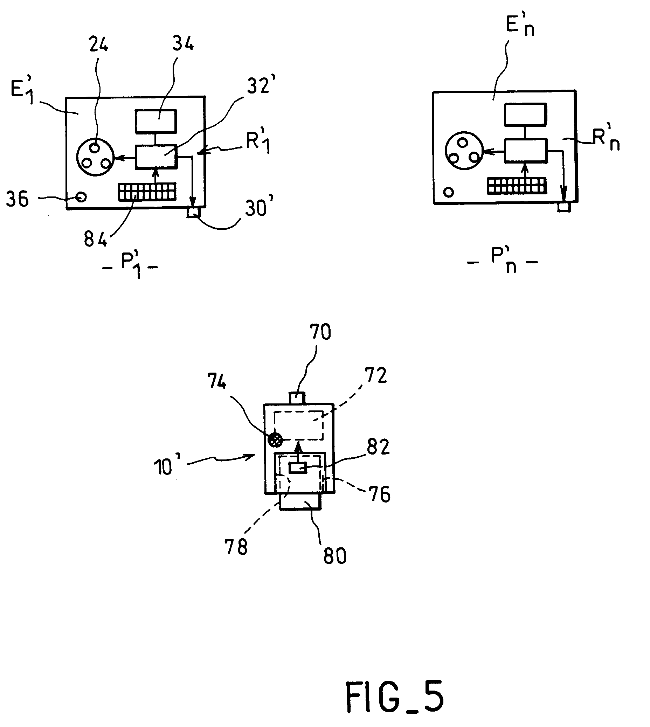

Dans le mode de réalisation simplifié représenté sur la figure 5 qui permet seulement la surveillance des équipements, l'installation comprend au moins un boîtier de surveillance à distance 10' et une pluralité d'équipements à surveiller E'1 à E'n installés respectivement dans les locaux P'1.....P'n. Le boîtier de surveillance 10' est simplifié par rapport au boîtier de surveillance et de commande 10. Il comprend essentiellement un récepteur de signaux électromagnétiques, de préférence de signaux infrarouges, ce récepteur étant référencé 70. Le récepteur 70 est relié à des circuits internes au boîtier qui portent la référence générale 72. Ces circuits 72 seront explicités ultérieurement en référence au schéma de la figure 3. Le boîtier comporte également un dispositif d'alarme 74 qui peut être optique, à vibration ou sonore. De préférence, le boîtier définit également un lecteur de cartes 76, ce lecteur comportant essentiellement une fente d'introduction de cartes 78 et, dans le cas où la carte 80 est du type à mémoire électronique, un connecteur 82 qui est relié au circuit 72. Bien entendu, la carte pourrait par exemple être du type magnétique, le lecteur étant bien sûr adapté. La carte comporte essentiellement dans sa mémoire une information d'identification de chaque personne susceptible de gérer l'installation. Lorsque la carte est introduite dans le lecteur 76, le circuit 72 lit l'information d'identification de son détenteur, cette information étant stockée dans la partie mémoire du circuit 72. Comme on l'expliquera ultérieurement, cela permet d'associer à chaque ensemble d'informations de fonctionnement d'un équipement E'i l'information relative à la personne qui a programmé l'équipement.

Chaque équipement E' comporte par exemple une pompe 24 associée à des circuits de commande et de traitement 32' et à une mémoire 34, cet ensemble formant le dispositif récepteur R'i. Pour commander la pompe 24, chaque équipement comporte un organe d'entrée d'informations, par exemple un clavier 84 qui permet d'entrer les instructions de commande du fonctionnement de la pompe 24. Lorsque le préposé a fini d'entrer les informations de commande du fonctionnement de la pompe 24, le circuit de traitement 32' commande un émetteur, par exemple infrarouge, 30' pour que celui-ci émette en direction du récepteur 70 du dispositif de surveillance 10' des signaux correspondants à l'ensemble de la séquence d'informations de commande préalablement introduite par le préposé.

L'utilisation de l'installation de surveillance à distance est la suivante : lorsque le préposé veut programmer un équipement, par exemple l'équipement E'1, celui-ci insère sa carte 80 dans le boîtier du dispositif portatif 10' et il introduit à l'aide du clavier 84 les instructions de commande du fonctionnement de la pompe 24. Ces instructions sont stockées dans la mémoire 34 et traitées par les circuits de traitement 32'. Lorsque le préposé a validé cette séquence d'instructions, le dispositif de traitement 32' commande l'émetteur infra-rouge 30' pour que celui-ci émette un signal représentatif des différentes instructions entrées et d'une information d'identification de l'équipement. Le dispositif portatif 10' est disposé de telle manière que son récepteur 70 reçoive ces signaux. Le circuit de traitement 72 convertit ces signaux en données numériques qui sont stockées dans une partie de la mémoire du circuit 72. Ces informations stockées dans la mémoire sont complétées par l'information d'identification lue dans la carte 80 de celui-ci. On obtient ainsi, après commande successive de plusieurs équipements E'i dans la mémoire du dispositif portatif 10', des ensembles d'informations comportant l'identification de l'équipement, l'identification du préposé et les instructions de commande de fonctionnement de l'équipement correspondant. Le circuit 72 comporte des éléments analogues à ceux qui sont représentés sur la figure 3 pour élaborer une information de temps écoulé à partir de l'introduction de ces données dans le dispositif portatif, ce qui correspondant au début du fonctionnement de l'équipement correspondant et des moyens pour comparer le temps écoulé à partir de cette initialisation à l'information représentative de la durée programmée de fonctionnement de l'équipement. Les circuits 72 comportent des composants qui commandent le dispositif d'alarme 74 lorsque la durée de fonctionnement est atteinte ou éventuellement, comme on l'a déjà expliqué en liaison avec les figures précédentes, qui commandent l'émission d'un signal de pré-alarme puis d'un signal d'alarme.

Il faut ajouter que, dans le cas du dispositif portatif 10 du premier mode de mise en oeuvre décrit en liaison avec les figures 1 à 4, il est également possible de prévoir que ce dispositif portatif comporte intégré dans son boîtier un lecteur de carte magnétique ou électronique dans lequel le préposé doit introduire sa carte pour permette le fonctionnement du dispositif de commande et de surveillance 10.

## Revendications

1. Installation de surveillance à distance d'une pluralité d'équipements commandables, chaque équipement comprenant des moyens de commande activés en réponse à une séquence d'informations de commande incluant une information de durée de fonctionnement, caractérisée en ce qu'elle comprend un dispositif portatif de commande et/ou surveillance (10) détenu par le préposé à la surveillance comportant :
. des moyens de mémorisation pour mémoriser au moins une partie des informations de commande relatives à chaque équipement en relation avec une information d'identification dudit équipement, lesdites informations mémorisées comprenant au moins l'information représentative de la durée commandée de fonctionnement dudit équipement,
. des moyens pour élaborer une information de temps,
. des moyens pour initialiser l'information de temps en relation avec la commande du début de fonctionnement dudit équipement et des moyens de mesure de temps par quoi une information de temps écoulé est obtenue, et
. des moyens pour comparer ladite durée de fonctionnement à l'information de temps écoulé et pour émettre un signal d'alarme en fonction du résultat de ladite comparaison.

2. Installation de surveillance à distance selon la revendication 1, caractérisée en ce qu'elle comprend en outre des moyens pour détecter la réception d'une information spécifique et des moyens pour n'autoriser l'introduction d'une séquence d'informations de fonctionnement dans un équipement qu'en réponse à la détection de ladite information spécifique.

3. Installation de surveillance à distance selon l'une quelconque des revendications 1 et 2, caractérisée en ce que les moyens de commande de chaque équipement comprennent en outre des moyens pour transmettre la séquence d'informations de commande de fonctionnement et en ce que ledit dispositif portatif comprend des moyens pour recevoir ladite séquence d'informations et pour stocker dans les moyens de mémorisation ladite séquence d'informations.

4. Installation de surveillance à distance selon l'une quelconque des revendications 1 et 2, caractérisée en ce qu'elle comprend en outre :
- une pluralité de dispositifs récepteurs (Ri) desdites informations de commande, chaque dispositif récepteur étant monté sur un desdits équipements,
ledit dispositif portatif de commande comprenant en outre :
. des moyens (40) pour élaborer ladite séquence d'informations de commande de fonctionnement d'un équipement ; et
- des moyens (12, 50) pour transmettre vers un dispositif récepteur ladite séquence d'informations de commande, chaque dispositif récepteur comportant :
. des moyens (30) de réception desdites informations de commande transmises, et
. des moyens (28) pour commander ledit équipement en fonction desdites informations.

5. Installation selon la revendication 3, caractérisée en ce que ladite séquence d'informations de commande comprend en outre une information d'identification de l'équipement commandé et en ce que chaque dispositif récepteur (Ri) comprend en outre des moyens (34) de mémorisation d'une information d'identification de l'équipement sur lequel il est monté, des moyens (32) pour comparer l'information d'identification mémorisée à l'information d'identification reçue et des moyens pour n'autoriser la mise en oeuvre d'au moins certaines des autres informations que s'il y a correspondance entre les deux informations d'identification.

6. Installation selon la revendication 4, caractérisée en ce que ladite séquence d'instructions comprend en outre une information d'identification du dispositif portatif de commande (10) et en ce que chaque dispositif récepteur (Ri) comprend des moyens (34) pour mémoriser l'information d'identification du dispositif portatif de commande, des moyens (32) pour comparer l'information d'identification mémorisée à l'information d'identification reçue ultérieurement et des moyens pour n'exécuter au moins certaines desdites informations de commande que s'il y a correspondance entre les deux informations d'identification.

7. Installation selon l'une quelconque des revendications 4 à 6, caractérisée en ce que chaque dispositif récepteur (Ri) comprend en outre des moyens émetteurs (30') pour retransmettre au moins une partie de la séquence d'informations de commande reçue, et en ce que ledit dispositif portatif de commande comporte des moyens de réception (12, 50) des informations réémises, des moyens (40) pour comparer les informations réémises aux informations émises, et des moyens pour émettre un signal d'acquittement si les informations émises et les informations réémises correspondent entre elles.

8. Installation selon la revendication 7, caractérisée en ce que ledit signal d'acquittement reçu par ledit dispositif portatif de commande initialise les moyens d'élaboration de l'information de temps (54) relative à l'équipement ayant transmis le signal d'acquittement.

9. Installation selon l'une quelconque des revendications 4 à 8, caractérisée en ce que lesdites informations sont émises sous forme d'ondes électromagnétiques.

10. Installation selon la revendication 9, caractérisée en ce que lesdites informations sont transmises sous forme d'un signal infrarouge.

11. Installation selon l'une quelconque des revendications 9 et 10, caractérisée en ce que ledit dispositif portatif de commande comprend un boîtier comportant un clavier (18), un dispositif d'affichage (16), des moyens émetteurs (12, 50) de l'onde électromagnétique et des circuits électroniques pour élaborer lesdites informations à partir des données entrées à l'aide du clavier, pour mémoriser lesdites informations et pour commander les moyens émetteurs de l'onde électromagnétique en fonction des informations élaborées.

12. Installation selon l'une quelconque des revendications 9 et 10, caractérisée en ce que chaque dispositif récepteur (Ri) comporte en outre des moyens (32, 30') pour transmettre des informations relatives au fonctionnement effectif de l'équipement (Ei) sur lequel est monté ledit dispositif récepteur, et en ce que ladite installation comprend en outre au moins une borne (Bi) apte à recevoir lesdites informations de fonctionnement effectif et à les transmettre vers un centre de surveillance (60, 62).

13. Installation selon l'une quelconque des revendications 4 et 8, caractérisée en ce que ledit dispositif portatif de commande (10) comporte en outre des moyens (40) pour programmer une information de durée de temps et en ce que lesdits moyens d'émission d'un signal d'alarme comprennent des moyens pour émettre un signal de pré-alarme à un instant qui précède la durée de fonctionnement d'au moins un équipement d'un temps égal à ladite durée de temps programmée.

14. Installation selon la revendication 5, caractérisée en ce qu'elle comprend une pluralité d'équipements (Ei), une pluralité de dispositifs récepteurs (Ri) et une pluralité de dispositifs portatifs de commande (10), chaque dispositif récepteur comportant des moyens pour mémoriser une pluralité d'informations d'identification de dispositifs portatifs de commande et des moyens pour autoriser l'exécution d'au moins certaines des informations de commande reçues si l'information d'identification du dispositif de commande ayant transmis lesdites informations est mémorisée dans ledit dispositif récepteur.

15. Installation selon l'une quelconque des revendications 1 à 14, caractérisée en ce que ledit dispositif portatif comporte en outre des moyens de lecture d'une information d'identification de l'utilisateur du dispositif portatif stockée dans un support amovible d'information et des moyens pour mémoriser ladite information d'identification de l'utilisateur avec la séquence d'informations de commande de fonctionnement d'un équipement.

## Claims

1. An installation for remote monitoring of a plurality of controllable equipments, each equipment comprising control means activated in response to a control information sequence including duration of operation information, characterised in that the installation comprises a portable control and/or monitoring device (10) held by the person responsible for monitoring and comprising:
• memorizing means for memorizing at least a portion of the control information relating to each equipment in association with information identifying said equipment, said memorized information including at least the information representative of the controlled operating duration of said equipment;
• means for generating time information;
• means for initializing the time information relative to the beginning of operation control of said equipment and time measuring means, thereby obtaining elapsed time information; and
• means for comparing said operating duration with the elapsed time information and for issuing an alarm signal as a function of the result of said comparison.

2. A remote monitoring installation according to claim 1, characterised in that it further includes means for detecting that a specific item of information has been received and means for authorizing a sequence of operating information to be input into an equipment only in response to said specific item of information.

3. A remote monitoring installation according to any one of claims 1 and 2, characterised in that the control means of each equipment further include means for transmitting the sequence of information for controlling operation and in that said portable device includes means for receiving said sequence of information and for storing said sequence of information in the memorizing means.

4. A remote monitoring installation according to any one of claims 1 and 2, characterised in that it further comprises:
- a plurality of receiver devices (Ri) for receiving said control information, each receiver device being mounted on a respective one of said equipments;
said portable control device further including:
• means (40) for generating said sequence of information for controlling the operation of an equipment; and
• means (12, 50) for transmitting said sequence of control information to a receiver device,
each receiver device comprising:
• means (30) for receiving said transmitted control information; and
• means (28) for controlling said equipment as a function of said information.

5. An installation according to claim 3, characterised in that said control information sequence further includes information identifying the controlled equipment, and in that each receiver device (Ri) further includes means (34) for memorizing information identifying the equipment on which it is mounted, means (32) for comparing the memorized identification information with the received identification information, and means for authorizing performance of at least some of the other information only if the two items of identification information correspond.

6. An installation according to claim 4, characterised in that said sequence of instructions further includes information identifying the portable control device (10), and in that each receiver device (Ri) includes means (34) for memorizing the identification information of the portable control device, means (32) for comparing the memorized identification information with the identification information received subsequently, and means for performing at least some of said control information only if the two items of identification information correspond.

7. An installation according to any one of claims 4 to 6, characterised in that each receiver device (Ri) further includes transmitter means (30') for re-transmitting at least a portion of the received control information sequence, and in that said portable control device includes means (12, 50) for receiving re-transmitted information, means (40) for comparing the re-transmitted information with the transmitted information, and means for transmitting an acknowledge signal if the transmitted information and the re-transmitted information correspond.

8. An installation according to claim 7, characterised in that said acknowledge signal received by said portable control device initializes the means for generating time information (54) relating to the equipment which transmitted the acknowledge signal.

9. An installation according to any one of claims 4 to 8, characterised in that said information is transmitted in the form of electro-magnetic waves.

10. An installation according to claim 9, characterised in that said information is transmitted in the form of an infrared signal.

11. An installation according to any one of claims 9 and 10, characterised in that said portable control device comprises a housing with a keypad (18), a display device (16), means (12, 50) for transmitting an electromagnetic wave, and electronic circuits for generating said information on the basis of data input via the keypad, for storing said information, and for controlling the means for transmitting the electromagnetic wave as a function of the generated information.

12. An installation according to any one of claims 9 and 10, characterised in that each receiver device (Ri) further includes means (32, 30') for transmitting information relating to the actual operation of the equipment (Ei) on which said receiver device is mounted, and in that said installation further includes at least one terminal (Bi) suitable for receiving said information about actual operation and for forwarding it to a monitoring center (60, 62).

13. An installation according to any one of claims 4 and 8, characterised in that said portable control device (10) further includes means (40) for programming time duration information, and in that said means for transmitting an alarm signal includes means for transmitting a pre-alarm signal at an instant that precedes the operating duration of at least one of the equipments by a length of time equal to said programmed time duration.

14. An installation according to claim 5, characterised in that it includes a plurality of equipment (Ei), a plurality of receiver devices (Ri), and a plurality of portable control devices (10), each receiver device including means for memorizing a plurality of items of portable control device identification information and means for authorizing performance of at least some received control information if the identification information of the control device that transmitted said information is stored in said receiver device.

15. An installation according to any one of claims 1 to 14, characterised in that said portable device further includes means for reading information identifying the user of the portable device and stored in a removable information medium, and means for memorizing said user identification information together with the sequence of information for controlling the operation of an equipment.

## Patentansprüche

1. Anordnung zur Fernüberwachung einer Vielzahl steuerbarer Geräte, von denen jedes Steuereinrichtungen aufweist, die in Abhängigkeit von einer Folge von Steuerinformationen, welche eine Information über die Betriebsdauer einschließen, aktiviert werden,
dadurch gekennzeichnet, daß sie eine von dem mit der Überwachung Beauftragten bewahrte tragbare Vorrichtung (10) zum Steuern und/oder Überwachen aufweist, die umfaßt:
- Speichereinrichtungen zum Speichern wenigstens eines Teils der sich auf jedes Gerät beziehenden Steuerinformationen in Verbindung mit einer Information zur Identifikation des genannten Gerätes, wobei die gespeicherten Informationen wenigstens die für die gesteuerte Betriebsdauer des genannten Gerätes repräsentative Information enthält,
- Einrichtungen zum Erstellen einer Zeitinformation,
- Einrichtungen zum Initialisieren der Zeitinformation in Verbindung mit dem Befehl zum Betriebsanfang des genannten Gerätes, und Einrichtungen zur Zeitmessung, wodurch eine Information über die abgelaufene Zeit erhalten wird, und
- Einrichtungen zum Vergleichen der genannten Betriebsdauer mit der Information über die abgelaufene Zeit und zum Senden eines Warnsignals in Abhängigkeit vom Ergebnis des genannten Vergleichs.

2. Anordnung zur Fernüberwachung nach Anspruch 1, dadurch gekennzeichnet, daß sie außerdem Einrichtungen zum Feststellen des Empfangs einer speziellen Information und Einrichtungen zum Zulassen der Eingabe einer Folge von Betriebsinformationen in ein Gerät nur in Abhängigkeit von der Feststellung der genannten speziellen Information aufweist.

3. Anordnung zur Fernüberwachung nach einem der Ansprüche 1 und 2,
dadurch gekennzeichnet, daß die Einrichtungen zum Steuern jedes Gerätes außerdem Einrichtungen zum Übertragen der Folge von Betriebssteuerinformationen aufweisen, und daß die genannte tragbare Vorrichtung Einrichtungen zum Empfangen der genannten Informationsfolge und zum Speichern der genannten Informationsfolge in den Speichereinrichtungen aufweist.

4. Anordnung zur Fernüberwachung nach einem der Ansprüche 1 und 2,
dadurch gekennzeichnet, daß sie außerdem umfaßt:
- eine Vielzahl von Vorrichtungen (Ri) zum Empfangen der genannten Steuerinformationen, wobei jede Empfangsvorrichtung auf einem der genannten Geräte angeordnet ist, daß die tragbare Steuervorrichtung außerdem umfaßt:
- Einrichtungen (40) zum Erstellen der genannten Folge von Steuerinformationen für den Betrieb eines Gerätes, und
- Einrichtungen (12, 50) zum Übertragen der genannten Folge von Steuerinformationen an eine Empfangsvorrichtung, wobei jede Empfangsvorrichtung umfaßt:
- Einrichtungen (30) zum Empfangen der übertragenen genannten Steuerinformationen, und
- Einrichtungen (28) zum Steuern des genannten Gerätes in Abhängigkeit von den genannten Informationen.

5. Anordnung nach Anspruch 3,
dadurch gekennzeichnet, daß die genannte Folge von Steuerinformationen außerdem eine Information zur Identifikation des gesteuerten Gerätes enthält, und daß jede Empfangsvorrichtung (Ri) außerdem Einrichtungen (34) zum Speichern einer Information zur Identifikation des Gerätes, auf dem sie angeordnet ist, aufweist, Einrichtungen (32) zum Vergleichen der gespeicherten Identifikationsinformation mit der empfangenen Identifikationsinformation, und Einrichtungen zum Zulassen der Ausführung wenigstens bestimmter weiterer Informationen nur, wenn sich die beiden Identifikationsinformationen entsprechen.

6. Anordnung nach Anspruch 4,
dadurch gekennzeichnet, daß die genannte Befehlsfolge außerdem eine Information zur Identifikation der tragbaren Steuervorrichtung (10) enthält, und daß jede Empfangsvorrichtung (Ri) Einrichtungen (34) zum Speichern der Information zur Identifikation der tragbaren Steuervorrichtung aufweist, Einrichtungen (32) zum Vergleichen der gespeicherten Identifikationsinformation mit der später empfangenen Identifikationsinformation, und Einrichtungen, die wenigstens bestimmte der genannten Steuerinformationen nur dann ausführen, wenn sich die beiden Identifikationsinformationen entsprechen.

7. Anordnung nach einem der Ansprüche 4 bis 6,
dadurch gekennzeichnet, daß jede Empfangsvorrichtung (Ri) außerdem Sendeeinrichtungen (30') zum Weiterleiten wenigstens eines Teils der empfangenen Steuerinformationsfolge aufweist, und daß die genannte tragbare Steuervorrichtung Einrichtungen (12, 50) zum Empfangen der wiedergesandten Informationen aufweist, Einrichtungen (40) zum Vergleichen der wiedergesandten Informationen mit den gesendeten Informationen und Einrichtungen zum Senden eines Quittungssignals, wenn die gesendeten Informationen und die wiedergesandten Informationen einander entsprechen.

8. Anordnung nach Anspruch 7,
dadurch gekennzeichnet, daß das von der tragbaren Steuervorrichtung empfangene genannte Quittungssignal die Einrichtungen (54) zum Erstellen der Zeitinformation bezüglich des Gerätes initialisiert, welches das Quittungssignal übertragen hat.

9. Anordnung nach einem der Ansprüche 4 bis 8,
dadurch gekennzeichnet, daß die genannten Informationen in Form elektromagnetischer Wellen gesendet werden.

10. Anordnung nach Anspruch 9,
dadurch gekennzeichnet, daß die genannten Informationen in Form eines Infrarot-Signals gesendet werden.

11. Anordnung nach einem der Ansprüche 9 und 10,
dadurch gekennzeichnet, daß die genannte tragbare Steuervorrichtung ein Gehäuse aufweist mit einer Tastatur (18), einer Anzeigevorrichtung (16), Sendern (12, 50) der elektromagnetischen Welle und mit elektronischen Schaltungsanordnungen zum Erstellen der genannten Informationen anhand der mit der Tastatur eingegebenen Daten, zum Speichern der genannten Informationen und zum Steuern der Sender der elektromagnetischen Welle in Abhängigkeit von den erstellten Informationen.

12. Anordnung nach einem der Ansprüche 9 und 10,
dadurch gekennzeichnet, daß jede Empfangsvorrichtung (Ri) außerdem Einrichtungen (32, 30') zum Übertragen von sich auf den effektiven Betrieb des Gerätes (Ei), auf dem die genannte Empfangsvorrichtung angeordnet ist, beziehenden Informationen, und daß die genannte Anordnung außerdem wenigstens einen Anschluß (Bi) aufweist, der die genannten Informationen über den effektiven Betrieb zu empfangen und sie zu einer Überwachungszentrale (60, 62) weiterzuleiten vermag.

13. Anordnung nach einem der Ansprüche 4 und 8,
dadurch gekennzeichnet, daß die genannte tragbare Steuervorrichtung (10) außerdem Einrichtungen (40) zum Programmieren einer Zeitdauer-Information aufweist, und daß die genannten Sender eines Warnsignals Einrichtungen aufweisen, die ein Vorwarnsignal zu einem Zeitpunkt senden, welcher der Betriebsdauer wenigstens eines Gerätes um eine Zeit vorausgeht, die der programmierten genannten Zeitdauer gleich ist.

14. Anordnung nach Anspruch 5,
dadurch gekennzeichnet, daß sie eine Vielzahl Geräte (Ei), eine Vielzahl Empfangsvorrichtungen (Ri) und eine Vielzahl tragbarer Steuervorrichtungen (10) umfaßt, wobei jede Empfangsvorrichtung Einrichtungen zum Speichern einer Vielzahl Identifikationsinformationen zu tragbaren Steuervorrichtungen und Einrichtungen aufweist, welche die Ausführung wenigstens bestimmter der empfangenen Steuerinformationen zulassen, wenn die Identifikationsinformation der Steuervorrichtung, welche die genannten Informationen übertragen hat, in der genannten Empfangsvorrichtung gespeichert ist.

15. Anordnung nach einem der Ansprüche 1 bis 14,
dadurch gekennzeichnet, daß die genannte tragbare Vorrichtung außerdem Einrichtungen zum Lesen einer Identifikationsinformation zum Benutzer der tragbaren Vorrichtung umfaßt, die in einem wegnehmbaren Informationsträger gespeichert ist, und Einrichtungen zum Speichern der genannten Identifikationsinformation zum Benutzer mit der Folge von Steuerinformationen zum Betrieb eines Gerätes.
